# EUROPÄISCHE PATENTSCHRIFT

(11) **EP 1 203 573 B1**
(45) Veröffentlichungstag und Bekanntmachung des Hinweises auf die Patenterteilung: **29.12.2004**
(21) Anmeldenummer: 01125929.8
(22) Anmeldetag: 31.10.2001
(51) Int. Cl.: A61K 7/06

(54) **Haarpflegemittel**
Hair care compositions
Compositions pour soins de cheveux

(30) Priorität: 02.11.2000 DE 10054215
(43) Veröffentlichungstag der Anmeldung: 08.05.2002
(73) Patentinhaber: KPSS-Kao Professional Salon Services GmbH, 64297 Darmstadt (DE)
(72) Erfinder: Bräutigam, Ina, 64297 Darmstadt (DE)

(56) Entgegenhaltungen:
- EP-A- 0 668 071
- EP-A- 0 766 959
- WO-A-00/40211
- WO-A-00/40213

## Beschreibung

Die vorliegende Erfindung betrifft ein Mittel zum Behandeln von menschlichem Haar, das diesem verbesserte Verformbarkeit, insbesondere Frisierbarkeit, guten Halt und gleichzeitig ein mattes Aussehen verleiht.

Mittel zum Konditionieren von menschlichen Haaren sind seit langem bekannt. Sie enthalten in der Regel quaternäre Ammoniumverbindungen, die mindestens eine langkettige Alkyl- oder Alkenylgruppe aufweisen, und gegebenenfalls auch Polymere.
Solche Mittel werden üblicherweise als wäßrige Dispersionen bzw. Emulsionen, Mikroemulsionen, Gele oder auch in Aerosolform konfektioniert und als Haarspülungen, Kuren etc. eingesetzt.
Eine Übersicht über die bekannten Haarnachbehandlungsmittel und ihre Zusammensetzung findet sich in der Monographie von K. Schrader, Grundlagen und Rezepturen der Kosmetika, 2. Aufl. (1989), S. 722 bis 781, insbesondere S. 728 bis 737.
Diese bekannten Zusammensetzungen sollen dem Haar verbesserte Eigenschaften, insbesondere einen deutlich erhöhten Volumeneffekt, leichtere Naß- und Trockenkämmbarkeit, Glanz, einen vollen lockeren Griff und Spannkraft verleihen.

Die Erfindung geht hingegen von der Aufgabenstellung aus, ein Haarpflegemittel zu schaffen, das dem damit behandelten Haar nicht nur guten Halt und eine dauerhafte Form verleiht, es sozusagen modellierbar macht, sondern, im Gegensatz zu dem sonst erwünschten Glanz, dem Haar, insbesondere auch hellem Haar, ein mattes Aussehen vermitteln soll.

Diese Aufgabe wird gelöst durch ein Haarpflegemittel, das als Emulsion vorliegt und
a) 1 bis 15 Gew.-% mindestens eines natürlichen und/oder synthetischen Wachses;
b) 1 bis 15 Gew.-% mindestens eines C₁₂-C₂₄-Fettalkohols;
c) 1 bis 25 Gew.-% mindestens eines Glycerin-C₁₂-C₂₄-Fettsäureesters;
d) 1 bis 25 Gew.-% mindestens eines Emulgators mit einem HLB-Wert >6;
e) 0,25 bis 5 Gew.-% mindestens eines Titandioxid-Pigments;
f) 2,5 bis 30 Gew.-% eines nichtionischen Polymeren, und
g) Wasser enthält.

Dieses Mittel wird, vorzugsweise nach einer Haarwäsche, gegebenenfalls im Anschluß an eine zuvor durchgeführte Haarfärbung oder Dauerwellung, auf das Haar aufgebracht, vorzugsweise in dieses einmassiert und nach der Einwirkung nicht entfernt.
Es handelt sich also um sogenannte "Leave on"-Produkte, im Gegensatz zu sogenannten "Rinse off"-Produkten, die nach einer kurzen Einwirkungszeit wieder aus dem Haar ausgespült werden.

Geeignete Titandioxid-Pigmente, die in den erfindungsgemäß zur Haarbehandlung verwendeten Mitteln in einer Menge von etwa 0,25 bis etwa 5, insbesondere etwa 0,1 bis etwa 4 Gew.-%, bezogen auf die Gesamtzusammensetzung des Mittels eingesetzt werden, sind an sich bekannt.

Vorzugsweise weisen dabei 90% der Teilchen einen Teilchendurchmesser im Bereich von etwa 0,05 bis 1 Mikron auf.

Als natürliche bzw. synthetische Wachse finden beispielsweise pflanzliche Wachse wie Candelillawachs, Carnaubawachs, Japanwachs, Korkwachs, Guarumawachs, Reiskeimölwachs, Zuckerrohrwachs und Montanwachs, tierische Wachse wie Bienenwachs, Schellackwachs, Walrat, Lanolin (Wollwachs), und Bürzelfett, Mineralwachse wie Ceresin, Ozokerit, Petrowachse wie Petrolatum, Paraffinwachse, Mikrowachse, chemisch modifizierte Wachse wie Montanesterwachse, Sasolwachse, hydr. Jojobawachse und synthetische Wachse wie Polyethylenwachse Verwendung, vorzugsweise in einer Menge von etwa 2,5 bis 10 Gew.-%.

Die C₁₂-C₂₄-Fettalkohole, insbesondere Laurylalkohol, Myristylalkohol, Cetylalkohol, Stearylalkohol und Cetylstearylalkohol, sind vorzugsweise ebenfalls in einer Menge von etwa 2,5 bis 10 Gew.-% anwesend.

Als Glycerin-C₁₂-C₂₄-fettsäureester werden insbesondere Cocoglyceride eingesetzt, jedoch sind prinzipiell auch Mono-, Di- und Triglyceride anderer Fettsäuren geeignet, vorzugsweise C₁₂-C₁₈-Fettsäureglyceride in einer Menge von insgesamt 5 bis 15 Gew.-%.

Die erfindungsgemäße Emulsion liegt vorzugsweise in Form einer Öl-in-Wasser-Emulsion vor, obwohl grundsätzlich auch andere Emulisonstypen wie Wasser-in-Öl-Emulsionen und Mischformen, sogenannte W/O/W-Emulsionen, möglich sind.
Essentiell ist die Anwesenheit mindestens eines Emulgators mit einem HLB-Wert von mindestens 6, insbesondere mindestens 8, vorzugsweise einem nichtionischen Emulgator.
Diese Emulgatoren sind dem Fachmann wohlbekannt; es wird hierzu, beispielhaft, auf K. Schrader, Grundlagen und Rezepturen der Kosmetika, 2. Aufl. (1989, Hüthig Buch Verlag), S. 390 bis 407, sowie die Aufzählung bei H.P. Fiedler, Lexikon der Hilfsstoffe für Pharmazie, Kosmetik und angrenzende Gebiete, 3. Aufl. (1989), S. 50 bis 55, verwiesen.

Es können jedoch auch ionische Emulgatoren eingesetzt werden, vorzugsweise im Gemisch mit nichtionischen Emulgatoren wie beispielsweise C₁₂-C₁₈-Fettalkoholethoxylate mit C₁₂-C₁₈-Alkyl(ether)sulfaten.
Der Anteil an Emulgatoren in den erfindungsgemäßen Zusammensetzungen beträgt vorzugsweise etwa 2 bis 20, insbesondere etwa 5 bis 15 Gew.-%.

Das nichtionische Polymer liegt vorzugsweise in einer Menge von etwa 5 bis 25, insbesondere etwa 7,5 bis 20 Gew.-% der Gesamtzusammensetzung vor. Seine Menge ist natürlich abhängig von der Art des Polymeren.
Bevorzugte nichtionische Polymere sind Polyethylenglykole und Polypropylenglykole, insbesondere in einem Molekulargewichtsbereich zwischen etwa 1000 und etwa 50000, vorzugsweise etwa 2000 bis etwa 25000.
Für diese Produkte liegt der bevorzugte Anteil bei etwa 10 bis etwa 20 Gew.-%. Besonders bevorzugt sind PEG-20, PEG-32, PEG-40, PEG-55, PEG-60, PPG-20, PPG-26, PPG-30 und/oder PPG-34.

Weitere geeignete nichtionische Polymere sind beispielsweise alkohol- und/oder wasserlösliche Vinylpyrrolidon-Polymere wie ein Vinylpyrrolidon-Homopolymerisat oder -Copolymerisat, insbesondere mit Vinylacetat.
Geeignete Vinylpyrrolidon-Polymere sind z.B. die unter dem Handelsnamen "Luviskol® " bekannten Produkte, beispielsweise die Homopolymerisate "Luviskol® K 30, K 60 und K 90" sowie die wasser- bzw. alkohollöslichen Copolymerisate aus Vinylpyrrolidon und Vinylacetat, die unter dem Handelsnamen "Luviskol® VA 55 bzw. VA 64" von der BASF AG vertrieben werden.
Weitere geeignete nichtionische Polymere sind Vinylpyrrolidon/Vinylacetat/Vinylpropionat-Copolymere wie "Luviskol® VAP 343", Vinylpyrrolidon/(Meth) Acrylsäureester-Copolymere sowie Chitosan-Derivate. Deren Anteil liegt bei etwa 2,5 bis 10 Gew.-% der Gesamt-Emulsion.

Die erfindungsgemäßen Mittel können zusätzliche weitere haarkonditionierende Wirkstoffe enthalten.

Geeignete haarkonditionierende Wirkstoffe, deren Menge in den erfindungsgemäß verwendeten Mitteln üblicherweise bei etwa 0,25 bis etwa 15, insbesondere 0,5 bis etwa 10, vorzugsweise etwa 0,75 bis 7,5 Gew.-%, bezogen auf die Gesamtmenge des Mittels, liegen kann, sind beispielsweise kationische Tenside, vor allem quaternäre Ammoniumverbindungen.

Geeignete langkettige quaternäre Ammoniumverbindungen, die allein oder im Gemisch miteinander eingesetzt werden können, sind beispielsweise Cetyltrimethylammoniumchlorid, Dimethyldicetylammoniumchlorid, Trimethylcetylammoniumbromid, Dimethyldistearylammoniumchlorid, Stearyltrimethylammoniumchlorid, Dimethylstearylbenzylammoniumchlorid, Benzyltetradecylodimethylammoniumchlorid, Dimethyl- dihydriertes-Talgammoniumchlorid, Laurylpyridiniumchlorid, Lauryldimethylbenzylammoniumchlorid, Behenyltrimethylammoniumchlorid, Lauryltrimethylammoniumchlorid, Tris-(oligooxyethyl)alkylammonium-phosphat, Cetyl-pyridiniumchlorid, etc.
Gut geeignet sind auch die in der EP-A 472 107 geoffenbarten quaternären Ammoniumsalze.
Im Prinzip sind alle quaternären Ammoniumverbindungen, wie sie im jeweils gültigen "CTFA International Cosmetic Ingredient Dictionary" unter dem Trivialnamen "Quaternium" aufgeführt sind, einsetzbar.

Ihr Anteil liegt vorzugsweise bei etwa 0,25 bis 10, insbesondere etwa 0,5 bis 7,5 Gew.-%, berechnet auf die Gesamtzusammensetzung.

Besonders geeignete langkettige quaternäre Ammoniumverbindungen sind Esterquats der allgemeinen Formel (I) 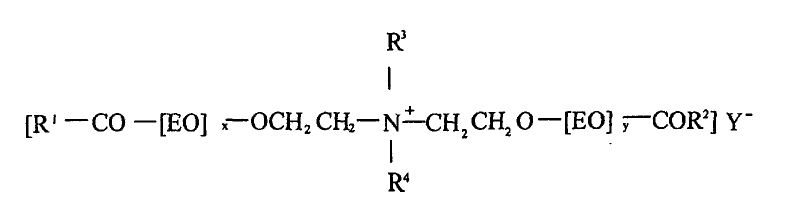 in der R¹ und R² für eine gegebenenfalls hydroxysubstituierte C₈-C₂₂-Alkyl- oder Alkenylgruppe, R³ und R⁴ für eine C₁-C₃-Alkylgruppe oder eine Gruppe -CH₂-CH₂-O-[EO]_{z}H sowie x,y und z für 0 bis 5 und Y⁻ für ein Anion stehen.

Ein besonders bevorzugtes Esterquat ist im Rahmen der Erfindung eine solche, in der die Reste R¹ und R² jeweils eine Oleylgruppe oder eine C₁₂-C₁₈-Alkylgruppe, der Rest R³ eine Methylgruppe und der Rest R⁴ eine Gruppe -CH₂-CH₂-0-[E0]_{z}-H bedeuten.

Das Anion Y⁻ ist vorzugsweise ein Halogenid wie Cl⁻ oder Br⁻, ein niederes Alkylsulfat, z.B. Methosulfat und Ethosulfat, oder ein Alkylphosphat, jedoch können selbstverständlich auch andere Reste eingesetzt werden.

Diese Verbindungen sind an sich bekannt und beispielsweise unter den Handelsnamen "Schercoquat^{A}", "Dehyquart^{R}F30" und "Tetranyl^{R}" im Handel.

Der Einsatz dieser Verbindungen, in Haarpflegemitteln ist ebenfalls bereits bekannt und beispielsweise in der WO-A 93/10748 und der WO-A 94/16677 beschrieben, wo sich jedoch keinerlei Hinweise auf die erfindungsgemäße Kombination und deren vorteilhafte Eigenschaften finden.

Ebenso geeignet sind "Amidoquats" der allgemeinen Formel 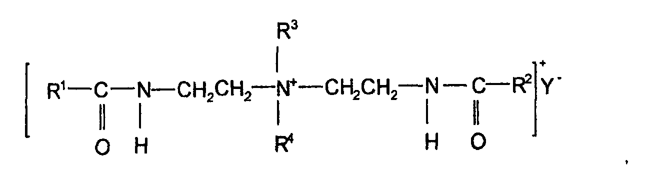 in der R¹ und R² jeweils für eine gegebenenfalls hydroxysubstituierte C₈-C₂₂-Alkyloder Alkenylgruppe, R³ und R⁴ für eine C₁-C₃-Alkylgruppe oder eine Gruppe -CH₂-CH₂-O-[EO]ₓ-H, sowie x für 0 bis 5, Y⁻ für ein Anion stehen.

Bevorzugte Reste R¹ und R² sind C₁₂-C₁₈-Alkyl- und Oleylreste, der Rest R³ eine Methylgruppe und der Rest R⁴ eine Gruppe -CH₂-CH₂-O[EO]ₓ-H, worin x 0 bis 3 ist; Y⁻ ist vorzugsweise ein Methosulfat-, Ethylsulfat-, Chlorid oder Phosphatanion.

Diese Verbindungen sind an sich bekannt und beispielsweise unter den Markenbezeichnungen "INCROQUAT® HO" oder "OCS" im Handel.

Weitere geeignete haarkonditionierende Wirkstoffe sind synthetische oder natürliche haarkonditionierende Polymere, vorzugsweise in einer Menge von 0,1 bis 10, insbesondere 0,25 bis 5 Gew.-% der Gesamtzusammensetzung.

Besonders bevorzugt sind hierbei die unter der CTFA-Bezeichnung "Polyquaternium" bekannten kationischen (Co-)Polymeren, jedoch können auch anionische und/oder amphotere Polymere, beispielsweise solche vom Typ "Amphomer^{R}", alleine oder im Gemisch verwendet werden.

Weitere haarkonditionierende Wirkstoffe sind lipophile, d.h. fett- und ölhaltige Substanzen, insbesondere natürliche Öle wie Avocadoöl, Cocosöl, Palmöl, Sesamöl, Erdnußöl, Spermöl, Sonnenblumenöl, Mandelöl, Pfirsichkernöl, Weizenkeimöl, Macadamianußöl, Nachtkerzenöl, Jojobaöl, Ricinusöl, oder auch Oliven-bzw. Sojaöl, Lanolin und dessen Derivate, ebenso Mineralöle wie Paraffinöl und Vaseline oder auch synthetische Öle wie Silikone.

Weitere geeignete hydrophobe Komponenten sind im Stearylalkohol und Fettsäureester wie Isopropylmyristat, -palmitat, -stearat und -isostearat, Oleyloleat, Isocetylstearat, Hexyllaurat, Dibutyladipat, Dioctyladipat, Myristylmyristat, Oleylerucat, Polyethylenglykol- und Polyglycerylfettsäureester wie PEG-7-glycerylcocoat, Cetylpalmitat, etc.

Diese hydrophoben Komponenten sind in den erfindungsgemäß verwendeten Zusammensetzungen vorzugsweise in einer Gesamtmenge von etwa 0,5 bis etwa 15, insbesondere etwa 1 bis 10, vor allem etwa 1,5 bis 7,5 Gew.-%, berechnet auf die Gesamtzusammensetzung, enthalten.

Ein weiterer bevorzugter Bestanteil der erfindungsgemäßen Zusammensetzung ist Grüner Tee-Extrakt.
Dieser Tee-Extrakt wird aus Blättern, Blattknospen und zarten Stielen des Teestrauchs, Camellia sinensis bzw. Camellia oleifera, durch wäßrige bzw. wäßrigalkoholische Extraktion und anschließende Sprühtrocknung erhalten.

Bei grünem Tee handelt es sich um die aus den Arten Thea sinensis bzw. Thea assamica gewonnenen, im Gegensatz zum schwarzen Tee nicht fermentierten Produkte.

Eine Übersicht über die biologische und pharmakologische Wirkung grünen Tees sowie seine Inhaltsstoffe findet sich z.B. in einem Artikel von A. Pistorius, SÖFW-Journal, 122. Jahrgang, No. 7/1996, S. 468-471, auf den Bezug genommen wird.

Der Gehalt an Extrakt aus grünem Tee in den erfindungsgemäßen Zusammensetzungen ist variabel. Er liegt vorzugsweise bei 0,01 bis 10, vorzugsweise 0,05 bis 5, insbesondere 0,25 bis 2,5 Gew.-%, berechnet auf die Gesamtzusammensetzung des Mittels und den pulverförmigen Extrakt.

Weitere geeignete haarkonditionierende Zusatzstoffe sind Ceramide der allgemeinen Formel 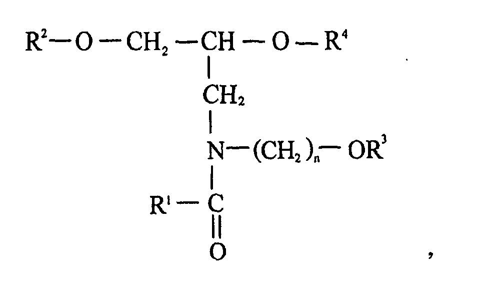 worin R¹ und R² gleiche oder verschiedene Alkyl- bzw. Alkenylreste mit 10 bis 22 Kohlenstoffatomen bedeuten, R³ für Wasserstoff oder eine Methyl-, Ethyl-, n-Propyloder Isopropylgruppe steht, R⁴ Wasserstoff, eine Hydroxymethyl-,Hydroxyethyl-, Dihydroxyethyl- oder Dihydroxypropylgruppe, und n eine ganze Zahl von 1 bis 6 bedeuten, insbesondere der Art, wie es aus der EP 227 994 A1 und der WO-A 96/37462 bekannt ist, jedoch sind auch andere Ceramide, beispielsweise die aus der WO-A 97/15724 oder der EP 647 617 B1 bekannten Ceramide, geeignet.

Die bevorzugten Gruppen R¹ und R² sind C₁₂-C₁₈-Alkylreste; n ist eine Zahl von 1 bis 3, R³ bedeutet vorzugsweise Wasserstoff oder einen Methylrest, und R⁴ Wasserstoff oder einen Dihydroxypropylrest.
Besonders bevorzugt sind Verbindungen, in denen R¹ einen C₁₂-C₂₄-Alkylrest, insbesondere eine C₁₃H₂₇-Alkylgruppe, R² einen C₁₄C₁₈-Alkylrest, insbesondere eine C₁₆H₃₃-Alkylgruppe, R³ einen Methylrest, R⁴ eine 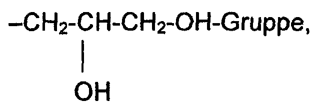 , und n 3 darstellen, oder eine Verbindung, wo R¹ für einen C₁₅-C₃₁-Alkylrest, R² für einen C₁₆H₃₃-Alkylrest, R³ und R⁴ für je ein Wasserstoffatom und n für 2 stehen.

Deren Menge in den erfindungsgemäß eingesetzten Haarbehandlungsmitteln liegt zweckmäßigerweise bei etwa 0,01 bis 10, vorzugsweise etwa 0,05 bis 7,5, insbesondere etwa 0,1 bis 5 Gew.-%, berechnet auf die Gesamtzusammensetzung.

Zusätzlich zu oder anstelle der genannten amphoteren bzw. zwitterionischen Tenside können die Haarpflegemittel mindestens ein C₁₂-C₁₈-Alkylamidopropyldimethyl- bzw. -diethylamin enthalten, beispielsweise Stearyl-, Oleyl- oder Cocoamidopropyldimethylamin.

Dessen Anteil liegt bei etwa 0,1 bis 10, vorzugsweise 0,25 bis 5 Gew.-% der Gesamtzusammensetzung.

Daneben sind natürlich auch anionische und/oder andere amphotere bzw. zwitterionische Tenside geeignet.

Die Zusammensetzungen können natürlich auch alle weiteren Stoffe enthalten, wie sie in Haarpflegeemulsionen grundsätzlich bekannt sind, wie Farbstoffe, Parfums, Komplexbildner, Eiweißhydrolysate, insbesondere kationisierte pflanzliche, Lecithine, Konservierungsmittel, Antioxidantien, UV-Absorber, Feuchthaltemittel wie Polyalkohole, Pflanzenextrakte, etc.
Besonders sinnvoll ist der Zusatz von Strukturverbesserern bzw. Verdickungsmitteln.

Geeignete Verdickungsmittel sind insbesondere Acrylsäure-Homo- und copolymerisate wie Acrylsäure/Acrylester- und Acrylsäure/Itaconsäureester-Copolymerisate, die gegebenenfalls auch vernetzt und/oder neutralisiert sind, z.B. die unter den CTFA-Bezeichnungen Acrylates/C₂₀-C₃₀-Alkyl Acrylates Crosspolymer, Acrylates Copolymer, Acrylates/Steareth Acrylates Copolymer, Acrylates/Acrylamides Copolymer, Acrylamides Copolymer, Acrylates/Ceteth Itaconate Copolymer, Acrylates/PVP Copolymer bekannten Produkte, sowie kationische Polymere, Cellulosederivate und sonstige Polysaccharide.
Diese Strukturverbesserer für Emulsionen sind an sich bereits bekannt, vgl. beispielsweise K. Schrader, I.c., S. 409-410, sowie Römpp Chemie Lexikon, 9. Aufl., S. 4890, und Fiedler, I.c., S. 32.
Der Anteil dieser Strukturverbesserer ist von deren Art und der Gesamtzusammensetzung der Emulsion abhängig und liegt bei etwa 0,05 bis 2,5, insbesondere 0,1 bis 1 Gew.-%.
Die folgenden Beispiele beschreiben die Erfindung im Detail.

### Beispiel 1

Es wurde ein **"Matt-Wachs**" folgender Zusammensetzung hergestellt:

| | |
|---|---|
| Cetylstearylalkohol | 4,00 (Gew.-%) |
| Ceteareth-3 | 9,00 |
| Laureth-23 | 0,20 |
| Candelillawachs | 3,00 |
| PEG-32 | 15,00 |
| Cocoglycerides (Gemisch aus Mono-, Di- und Triglyceriden des Cocosöls) | 9,00 |
| Natriumcetearylsulfat | 0,50 |
| Parfum | 0,10 |
| Grüner Tee-Extrakt | 0,50 |
| Konservierungsmittel | 0,30 |
| Eisenoxid (C.I.-No. 77,499) | 0,20 |
| Ti0₂-Pigment (C.I.-No. 77,891; mittl. Teilchendurchmesser: < 1 Mikron) | 3,00 |
| Acrylates / C₁₀-C₃₀-Alkyl Acrylate Crosspolymer | 0,15 |
| Aminomethylpropanol | 0,12 |
| Wasser | ad 100,00 |

An leicht strapazierten, gebleichten Menschenhaar-Strähnen wurde folgender Blindversuch durchgeführt:

In 5 Strähnen wurde nach der Haarwäsche mit einem handelsüblichen Shampoo in das noch feuchte Haar eine Zusammensetzung 1 entsprechend dem obigen Beispiel einmassiert.

Weitere 5 Strähnen wurden mit einer mit der Zusammensetzung 1 identischen Zusammensetzung 1A, die jedoch keine Pigmentteilchen enthielt, in identischer Weise behandelt.

Die Haarsträhnen wurden vor und nach dem Trocknen von jeweils 2 Friseuren nach der Präferenzmethode beurteilt.

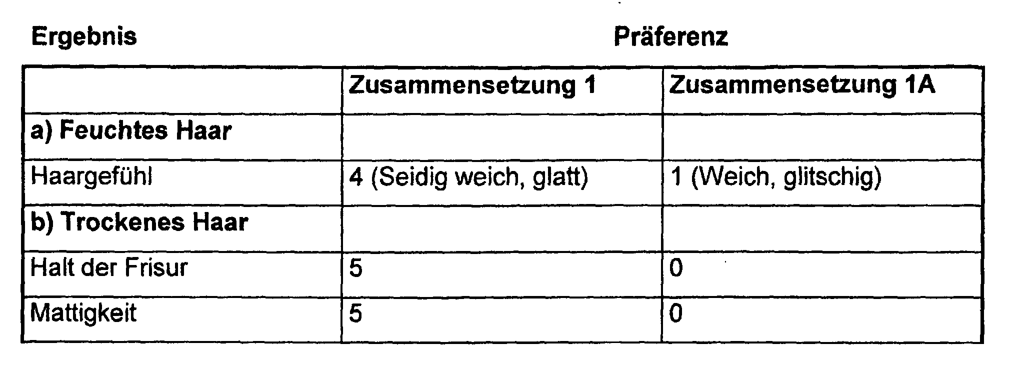

Dieses Ergebnis zeigt die überraschende Überlegenheit des erfindungsgemäßen Mittels, wobei auch die Tatsache als überraschend anzusehen ist, daß auch bei blondem (= gebleichtem) Haar ein ausgezeichneter mattierender Effekt erzielt wird.

### Beispiel 2

Es wurde ein **"Matt-Wachs**" folgender Zusammensetzung hergestellt:

| | |
|---|---|
| 1,2-Propylenglykol | 2,00 (Gew.-%) |
| Carbopol^{R}ETD 2020 (Acrylates/ | 0,50 |
| C₁₀-C₂₀-Alkyl acrylate crosspolymer) | |
| Cocoamidopropylbetain | 1,00 |
| Cocotroglycerides (Gemisch aus Mono-, Di- und Triglycerid) | 7,50 |
| PPG 20 | 12,00 |
| PEG-60 Hydriertes Ricinusöl | 0,50 |
| Ceteth-10 | 7,50 |
| NaOH (32%) | 0,25 |
| Parfum | 0,10 |
| Jojobawachs | 5,00 |
| Cetylalkohol | 4,50 |
| Mandelöl | 2,00 |
| Ti0₂-Pigment (90% mit durchschnittl. Teilchendurchmesser < 1µm) | 2,60 |
| Mica (C.I.-No. 77,019) | 0,10 |
| Konservierungsmittel | 0,35 |
| Wasser | ad 100,0 |

Dieses Produkt wurde auf trockenes Haar aufgebracht und führte zu einem matten Haar mit ausgezeichneter Frisierbarkeit und stabilem Halt der erzielten Frisur.

### Beispiel 3

Ein **"Matt-Wachs**" der folgenden Zusammensetzung:

| | |
|---|---|
| Tocopherylacetat | 0,5 (Gew.-%) |
| Mica/Ti0₂-Pigment (1:10; mittl. Teilchengröße: < 90%) | 2,5 |
| Carbopol^{R} ETD 2001 (Carbomer) | 0,5 |
| PEG-18 | 7,0 |
| Vinylpyrrolidon/Vinylacetat-Copolymer (Luviskol^{R} VA 551) | 5,5 |
| Glyceridgemisch (C₁₂-C₁₄-Mono-, Di- und Triglyceride) | 5,5 |
| Aminomethylpropanol | 0,4 |
| Cocoamidopropylbetain | 1,0 |
| C₁₂-C₁₄-Fettalkoholgemisch | 6,5 |
| PEG-60 Hydriertes Ricinusöl | 1,0 |
| Laureth-8 | 5,5 |
| Oleth-10 | 1,5 |
| Parfum | 0,3 |
| Benzophenone-4 | 0,3 |
| Konservierungsmittel | 0,3 |
| Wasser | ad 100,0 |

ergab nach dem Einmassieren in frisch gewaschenes, handtuchtrockenes Haar einen exzellenten Halt sowie ein attraktives mattes Aussehen des Haares.

## Patentansprüche

1. Haarpflegemittel auf Basis einer Emulsion, enthaltend
a) 1 bis 15 Gew.-% mindestens eines natürlichen und/oder synthetischen Wachses;
b) 1 bis 15 Gew.-% mindestens eines C₁₂-C₂₄-Fettalkohols;
c) 1 bis 25 Gew.-% mindestens eines Glycerin-C₁₂-C₂₄-Fettsäureesters;
d) 1 bis 25 Gew.-% mindestens eines Emulgators mit einem HLB-Wert >6;
e) 0,25 bis 5 Gew.-% mindestens eines Titandioxid-Pigments;
f) 2,5 bis 30 Gew.-% eines nichtionischen Polymeren, und
g) Wasser.

2. Mittel nach Anspruch 1, **dadurch gekennzeichnet, daß** 90 Gew.-% des Titandioxid-Pigments einen mittleren Teilchendurchmesser zwischen 0,05 und 1 Mikron aufweisen.

3. Mittel nach Anspruch 1 und/oder 2, enthaltend als Bestandteil f) etwa 5 bis 25 Gew.-% mindestens eines Polyethylenglykols und/oder Polypropylenglykols mit einem Molgewicht zwischen etwa 1000 und 50000.

4. Mittel nach einem oder mehreren der Ansprüche 1 bis 3, enthaltend zusätzlich ein Verdickungsmittel.

5. Mittel nach Anspruch 4, enthaltend als Verdickungsmittel 0,05 bis 2,5 Gew.-% eines gegebenenfalls vernetzten Acrylsäurehomo- oder copolymerisats.

## Claims

1. Hair care composition on the basis of an emulsion, comprising
a) 1 % to 15 % by weight of at least one natural and/or synthetic wax;
b) 1 % to 15 % by weight of at least one C₁₂-C₂₄-fatty alcohol;
c) 1 % to 25 % by weight of at least one glycerol-C₁₂-C₂₄-fatty acid ester;
d) 1 % to 25 % by weight of at least one emulsifier with an HLB-value of >6;
e) 0.25 % to 5 % by weight of at least one titanum dioxide pigment;
f) 2.5 % to 30 % by weight of a nonionic polymer, and
g) water.

2. Composition according to claim 1, wherein 90 % by weight of the titanum dioxide pigment has an average particle diameter ranging between 0.05 and 1 micron.

3. Composition according to claim 1 and/or 2, comprising as component f) about 5 % to 25 % by weight of at least one polyethylene glycol and/or polypropylene glycol with a molecular weight between about 1000 and 50,000.

4. Composition according to one or more of claims 1 to 3, additionally comprising a thickening agent.

5. Composition according to claim 4, comprising as thickening agent 0.05 % to 2.5 % by weight of an optionally cross-linked acrylic acid homo- or copolymer.

## Revendications

1. Agent de soins capillaires à base d'une émulsion, contenant
a) 1 à 15% en poids d'au moins une cire naturelle et/ou synthétique;
b) 1 à 15% en poids d'au moins un alcool gras en C₁₂ à C₂₄;
c) 1 à 25% en poids d'au moins un ester de glycérine et d'acide gras en C₁₂ à C₂₄;
d) 1 à 25% en poids d'au moins un émulsifiant avec une valeur HLB > 6;
e) 0,25 à 5% en poids d'au moins un pigment au dioxyde de titane;
f) 2,5 à 30% en poids d'un polymère non ionique, et
g) de l'eau.

2. Agent selon la revendication 1, **caractérisé en ce que** 90% du pigment au dioxyde de titane présentent un diamètre moyen de particule compris entre 0,05 et 1 micromètre.

3. Agent selon la revendication 1 et/ou 2, contenant comme composant f), environ 5 à 25% en poids d'au moins un polyéthylèneglycol et/ou polypropylèneglycol ayant une masse moléculaire comprise entre environ 1 000 et 50 000.

4. Agent selon l'une ou plusieurs des revendications 1 à 3, contenant en sus un épaississant.

5. Agent selon la revendication 4, contenant 0,05 à 2,5% en poids d'un homo- ou co-polymère de l'acide acrylique éventuellement réticulé, en tant qu'épaississant.
